# FASCICULE DE BREVET EUROPEEN

(11) **EP 0 493 221 B1**
(45) Date de publication et mention de la délivrance du brevet: **25.01.1995**
(21) Numéro de dépôt: 91403482.2
(22) Date de dépôt: 20.12.1991
(51) Int. Cl.: A61N 1/05

(54) **Sonde endocardiaque munie d'un organe de fixation active**
Endskardiumsonde mit einem aktiven Befestigungsteil
Endocardial lead provided with active fixation means

(30) Priorité: 27.12.1990 FR 9016294
(43) Date de publication de la demande: 01.07.1992
(73) Titulaire: ELA MEDICAL (Société anonyme), F-92541 Montrouge (FR)
(72) Inventeur: Bens, Jean-Luc, F-62400 Béthune (FR)
(74) Mandataire: Laget, Jean-Loup

(56) Documents cités:
- EP-A- 0 009 619
- EP-A- 0 149 431
- EP-A- 0 296 001
- FR-A- 2 310 775

## Description

La présente invention se rapporte aux sondes endocardiaques pour stimulateurs cardiaques du type dont l'extrémité distale est munie d'un organe de fixation active constitué par une mèche hélicoïdale qui s'enfonce dans le muscle cardiaque.

Pour la stimulation cardiaque ou pour recueillir des signaux électriques en provenance du coeur, il est connu d'employer des sondes endocardiaques, appelées aussi cathéters, qui sont constituées par un conducteur réalisé par un fil métallique conducteur enroulé en spirale de façon analogue à un ressort, ce fil conducteur en spirale étant enrobé dans une gaine souple chimiquement neutre vis-à-vis des fluides du corps humain, cette gaine étant par exemple réalisée en caoutchouc au silicone. Cette sonde comporte deux extrémités : l'une appelée proximale, qui est, dans le cadre d'un stimulateur, connectée à la source d'énergie électrique, l'autre appelée distale et qui est mise en contact avec la paroi interne du muscle cardiaque. Cette extrémité distale comporte une électrode conductrice reliée au fil conducteur enroulé. De telles sondes sont introduites à l'intérieur du coeur en passant par une veine, en général la veine céphalique.

Lorsque la sonde doit être placée dans l'oreillette dont les parois sont relativement lisses, il est avantageux de disposer à l'extrémité distale un organe de fixation active constitué par une mèche hélicoïdale en forme de tire-bouchon ou queue de cochon, cette mèche étant destinée à être enfoncée dans le tissu cardiaque par rotation.

Il est connu de disposer à l'extrémité distale une telle mèche qui fait saillie hors de cette extrémité. Lorsque cette dernière a été amenée, par le praticien, à l'endroit voulu à l'intérieur de l'oreillette, l'extrémité distale est fixée en position en faisant tourner la sonde elle-même. Une telle mèche hélicoïdale, qui fait saillie à l'extrémité distale de la sonde, présente l'inconvénient qu'elle risque de provoquer des blessures dans la veine ou de se piquer dans les valves cardiaques.

Il a alors été proposé de disposer à l'extrémité distale une mèche hélicoïdale rétractable, c'est-à-dire que cette mèche est mise en position escamotée à l'intérieur de l'extrémité distale lors de la mise en place de la sonde, cette dernière étant munie de moyens permettant de faire sortir la mèche en tournant lorsque la sonde est correctement placée. Pour obtenir ce mouvement simultané de sortie et de rotation de la mèche, on a proposé par exemple dans le brevet US 4 217 913 de disposer la mèche à l'intérieur de l'extrémité distale de la sonde et de la relier à un embout de commande cylindrique également logé dans la sonde. Cet embout comporte une fente destinée à coopérer avec un mandrin introduit dans la sonde pour la mise en rotation de la mèche par l'intermédiaire de l'embout. Lors de l'extraction de la sonde, la mèche hélicoïdale prend appui sur un monofilament qui traverse radialement la sonde et dont les extrémités sont thermosoudées à la surface externe de la sonde.

L'inconvénient de cette sonde est que le diamètre de sa tête est relativement important et qu'elle est compliquée à fabriquer. On peut ainsi constater que toutes les sondes endocardiaques connues comportant un tel organe de fixation rétractable ont au moins à leur extrémité distale un diamètre relativement important, ce qui constitue un inconvénient non négligeable lors de la mise en place de la sonde en passant par une veine, mais aussi pour la commande en rotation de l'organe de fixation hélicoïdale, du fait que des rétrécissements qui compriment localement la sonde dans le sens radial gênent aussi la rotation des moyens de commande de l'organe de fixation.

Le but de l'invention est de proposer une sonde munie d'un organe de fixation sous forme d'une mèche hélicoïdale et qui peut facilement être mise en place et commandée de l'extérieur. La sonde selon l'invention comporte par ailleurs un minimum de pièces destinées au guidage et à l'appui de la mèche hélicoïdale et la fabrication de la sonde est ainsi sensiblement simplifiée.

L'invention a pour objet une sonde endocardiaque du type comportant un enroulement en spirale, conducteur, disposé à l'intérieur d'une gaine en matériau souple, isolant et insensible aux fluides du corps, cet enroulement en spirale étant connecté électriquement à une électrode destinée à être mise en contact avec la paroi interne du coeur, ladite électrode étant creuse et traversée selon son axe par une mèche hélicoïdale destinée à fixer la sonde dans le muscle cardiaque, caractérisée en ce que le diamètre intérieur de l'électrode est inférieur au diamètre extérieur de la mèche hélicoïdale, en ce que la mèche présente un coefficient d'élasticité longitudinale suffisant pour être rétractable dans l'électrode avec déformation mécanique, et en ce que la mèche retrouve sensiblement sa forme initiale lors de son extraction de l'électrode.

Selon d'autres caractéristiques de l'invention :
- La mèche hélicoïdale est fabriquée en un alliage à base de nickel-titane ;
- l'extraction de la mèche hélicoïdale hors de l'électrode est effectuée par une rotation commandée par un organe de commande inséré dans la sonde, et la paroi interne de l'électrode présente une déformation en forme de cran sur lequel la mèche prend appui lors de son extraction.

L'invention sera maintenant décrite plus en détail en se référant au dessin annexé sur lequel :
La Fig. 1 est une coupe longitudinale de l'extrémité distale de la sonde montrant la mèche hélicoïdale dans sa position rétractée dans la sonde avant et lors de l'introduction de celle-ci dans le coeur ;
La Fig. 2 est une vue en coupe longitudinale de l'extrémité distale de la sonde montrant la mèche hélicoïdale au cours de son extraction de la sonde pour la fixation de celle-ci au muscle cardiaque par vissage.

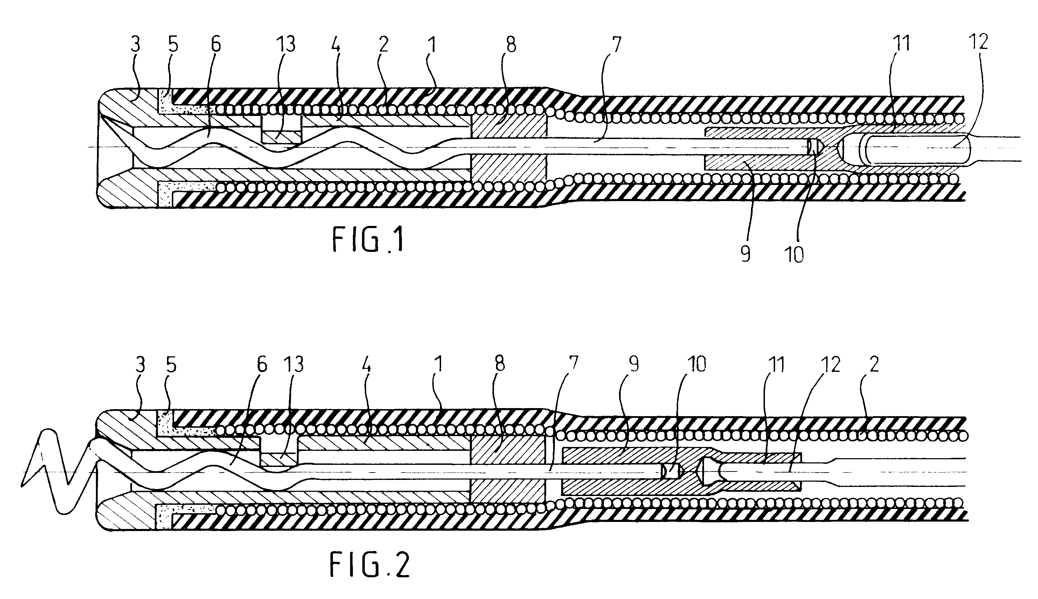

Les figures 1 et 2 montrent l'extrémité distale d'une sonde endocardiaque suivant l'invention. La sonde comporte une gaine 1 en un matériau tel qu'un élastomère silicone qui est souple, isolant et insensible aux fluides du corps.

La gaine 1 enveloppe un enroulement conducteur 2 formé en spirale. L'enroulement 2 est à son extrémité proximale relié à un générateur de signaux électriques constituant le stimulateur cardiaque (non représenté). L'extrémité distale de l'enroulement est reliée à une électrode creuse 3 destinée à venir en butée contre la paroi interne du muscle cardiaque.

Dans l'exemple illustré sur les figures, cette électrode est sensiblement annulaire et comporte un prolongement tubulaire 4 s'étendant vers l'intérieur de la sonde de manière à venir en contact avec un certain nombre de spires de l'enroulement 2 pour assurer le transfert à l'électrode des impulsions électriques en provenance du stimulateur.

L'électrode 3 est fixée à l'extrémité distale de la gaine 1 à l'aide d'un adhésif 5 intercalé entre ces deux éléments.

La sonde comporte à son extrémité distale un organe de fixation sous forme d'une mèche hélicoïdale 6 s'étendant coaxialement à l'électrode. La partie proximale de la mèche forme une tige rectiligne 7 qui s'étend à travers un joint d'étanchéité cylindrique 8 en un matériau isolant électriquement. L'extrémité de cette partie proximale est reliée à un embout de commande 9, également cylindrique.

L'extrémité libre de la partie formant tige 7 de la mèche 6 est insérée et fixée par serrage dans un alésage 10 de l'embout 9 qui peut être en un matériau métallique. La tige 7 peut naturellement être fixée à l'embout 9 d'une autre manière appropriée. L'extrémité opposée de l'embout 9 comporte une fente radiale 11 destinée à recevoir l'extrémité aplatie en tournevis d'un mandrin 12 qui est introduit dans la sonde pour commander la rotation de l'embout 9 et, avec celui-ci, de la mèche 6. Les autres moyens utilisés lors de la mise en place de la sonde et de la fixation de celle-ci ne seront pas décrits plus en détail étant donné qu'ils ne se rapportent pas directement à l'invention.

Le mandrin 12, qui ainsi constitue avec l'embout 9 un organe de commande en rotation de la mèche, est retiré de la sonde après la mise en place de celle-ci. Cet organe de commande peut selon une variante (non représentée) être constitué d'un embout en un matériau isolant relié à demeure à un second enroulement, par exemple fabriqué en acier inoxydable, et dans ce cas ce dernier reste dans la sonde après le vissage de la mèche.

La mèche hélicoïdale 6 est en un matériau présentant un grand coefficient d'élasticité longitudinale, comme par exemple un alliage métallique, de préférence à base de titane-nickel. Un tel alliage permet un allongement de l'ordre de 8%, comparativement à l'acier inoxydable dont l'allongement admis n'est que de 0,3 %, ce qui donne la possibilité d'obtenir avec l'alliage Ti-Ni des rayons de courbure extrêmement faibles.

La grande élasticité longitudinale d'un tel alliage permet la fabrication d'une mèche hélicoïdale 6 dont le diamètre extérieur est sensiblement supérieur au diamètre intérieur de l'électrode annulaire 3, mais qui peut néanmoins être rétracté dans l'électrode en subissant une déformation mécanique qui consiste en un allongement de la mèche avec réduction de son diamètre extérieur. La mèche est ainsi logée avec une certaine précontrainte à l'intérieur de l'électrode, ce qui fait qu'elle reprend sensiblement sa forme initiale dès qu'elle ressort de l'électrode tout en étant vissée dans le tissu du muscle cardiaque.

Il est préférable d'isoler électriquement la mèche 6, afin de ne stimuler le coeur que par l'électrode 3. Dans l'exemple décrit, la mèche 6 est isolée électriquement par un revêtement isolant (non représenté) qui peut être du téflon. Il suffit par ailleurs d'appliquer ce revêtement isolant sur la partie distale de la mèche.

Il est également possible de stimuler le coeur à la fois par l'électrode 3 et par la mèche 6 et seule une troisième possibilité consiste à utiliser la mèche métallique 6 comme électrode. Dans ce dernier cas, elle serait reliée directement à l'enroulement 2.

La sonde est pourvue de moyens de retenue et d'appui de la mèche hélicoïdale 6. Ces moyens comportent un cran 13 faisant saillie vers l'intérieur de la sonde à partir de la paroi interne de l'électrode. Ce cran 13 est obtenu par une déformation de la paroi de l'électrode.

Le cran 13 est d'un côté destiné à retenir la mèche en place dans une position escamotée à l'intérieur de l'électrode, comme cela est illustré sur la figure 1, et d'un autre côté à offrir un appui pour la mèche pour que celle-ci puisse être déplacée vers l'extérieur par un simple mouvement de rotation qui lui est imprimé à l'aide du mandrin 12.

A titre purement informatif, la mèche suivant l'invention peut présenter les caractéristiques mécaniques suivantes :
- allongement à la rupture 15 à 20%
- allongement admis 8%
- module de Young 9800 daN/mm²
- limite élastique 42 daN/mm²
- coefficient de Poisson 0,33
- résistance à la rupture 88 daN/mm²
Grâce à l'invention qui permet une réduction sensible du diamètre de la tête de la sonde, celle-ci peut facilement être introduite dans le coeur de sorte que l'extrémité distale de celle-ci prenne appui contre le muscle cardiaque à l'endroit souhaité. Ensuite la mèche hélicoïdale 6 est automatiquement sortie de l'électrode lorsqu'on la fait tourner à l'aide du mandrin 12 et elle reprend sa forme initiale au fur et à mesure qu'elle sort de l'électrode pour atteindre la position sortie illustrée sur la figure 2, tout en se vissant dans le muscle cardiaque. L'électrode est ainsi bien ancrée dans le coeur et se trouve constamment sollicitée vers la paroi interne de celui-ci, ce qui donne un bon contact pour la transmission des impulsions électriques.

## Revendications

1. Sonde endocardiaque comportant un enroulement en spirale (2), conducteur, disposé à l'intérieur d'une gaine (1) en matériau souple, isolant et insensible aux fluides du corps, cet enroulement en spirale (2) étant connecté électriquement à une électrode (3) destinée à être mise en contact avec la paroi interne du coeur, ladite électrode (3) étant creuse et traversée selon son axe par une mèche hélicoïdale rétractable (6) destinée à fixer la sonde dans le muscle cardiaque, caractérisée en ce que le diamètre intérieur de l'électrode (3) est inférieur au diamètre extérieur de la mèche hélicoïdale (6), en ce que la mèche (6) présente un coefficient d'élasticité longitudinale suffisant pour être rétractable dans l'électrode (3) avec déformation mécanique, et en ce que la mèche retrouve sensiblement sa forme initiale lors de son extraction de l'électrode.

2. Sonde suivant la revendication 1, caractérisée en ce que la mèche hélicoïdale (6) est fabriquée en un alliage métallique.

3. Sonde suivant la revendication 2, caractérisée en ce que l'alliage est un alliage à base de nickel-titane.

4. Sonde suivant une quelconque des revendications précédentes, caractérisée en ce que la mèche 6 est recouverte, au moins à sa partie distale, par un revêtement électriquement isolant.

5. Sonde suivant la revendication 4, caractérisée en ce que le revêtement est du téflon.

6. Sonde suivant la revendication 1 ou 2, dans laquelle l'extraction de la mèche hélicoïdale (6) hors de l'électrode (3) est effectuée par une rotation commandée par un organe de commande (9, 12) inséré dans la sonde, caractérisée en ce que la paroi interne de l'électrode (3) présente une déformation en forme de cran (13) sur lequel la mèche (6) prend appui lors de son extraction.

## Claims

1. An endocardial probe comprising a conductive, spiral winding (2) arranged inside a sheath (1) in a flexible, insulating material that is not sensitive to body fluids, said spiral winding (2) being electrically connected to an electrode (3) intended to be brought into contact with the inner wall of the heart, said electrode (3) being hollow and passed through along its axis by a retractable helical bore bit (6) intended to fasten the probe into the cardiac muscle, characterized in that the inside diameter of the electrode (3) is smaller than the outside diameter of the helical bore bit (6), and in that the bore bit (6) has a coefficient of longitudinal elasticity that is sufficient for it to be retractable into the electrode (3) with mechanical distortion, and in that the bore bit returns substantially to its initial shape when extracted from the electrode.

2. The probe as claimed in claim 1, characterized in that the helical bore bit (6) is manufactured in a metal alloy.

3. The probe as claimed in claim 2, characterized in that the alloy is an NiTi-based alloy.

4. The probe as claimed in any one of the preceding claims, characterized in that the bore bit (6) is covered, at least at its distal end, by an electrically insulating covering.

5. The probe as claimed in claim 4, characterized in that the covering is Teflon.

6. The probe as claimed in claim 1 or claim 2, wherein the helical bore bit (6) is extracted from the electrode (3) by means of a rotation commanded by a control device (9, 12) inserted into the probe, characterized in that the inner wall of the electrode (3) has a notch-shaped deformation (13) against which the bore bit (6) leans when it is being extracted.

## Patentansprüche

1. Endokardiumsonde, bestehend aus einer leitfähigen Spiralwicklung (2), die im Inneren einer aus einem biegsamen, isolierenden und gegen Körperflüssigkeiten unempfindlichen Material bestehenden Umhüllung (1) angeordnet ist, wobei die Spiralwicklung (2) elektrisch mit einer zum Berühren dar Herzinnenwand vorgesehenen Elektrode (3) verbunden ist, die Elektrode (3) hohl ist und entlang ihrer Achse ein zum Fixieren der Sonde im Herzmuskel vorgesehener, zurückziehbarer Schneckenbohrer (6) eingebracht ist, dadurch gekennzeichnet, daß der Innendurchmesser der Elektrode (3) kleiner als der Außendurchmesser des Schneckenbohrers (6) ist, daß der Bohrer (6) einen in Längsrichtung ausreichend großen Elastizitätskoeffizienten aufweist, um unter mechanischer Verformung in die Elektrode (3) zurückziehbar zu sein, und daß der Bohrer seine ursprüngliche Form beim Ausbringen aus der Elektrode leicht wieder annimmt.

2. Sonde nach Anspruch 1, dadurch gekennzeichnet, daß der Schneckenbohrer (6) aus einer Metall-Legierung besteht.

3. Sonde nach Anspruch 2, dadurch gekennzeichnet, daß die Legierung eine Nickel-Titan-Legierung ist.

4. Sonde nach eine der vorhergehenden Ansprüche, dadurch gekennzeichnet, daß zumindest der abliegende Teil des Bohrers (6) von einem elektrisch isolierenden Mantel umhüllt ist.

5. Sonde nach Anspruch 4, dadurch gekennzeichnet, daß der Mantel aus Teflon besteht.

6. Sonde nach Anspruch 1 oder 2, bei der das Ausbringen des Schneckenbohrers (6) aus der Elektrode (3) durch eine von einer in die Sonde eingefügten Steuereinrichtung (9, 12) gesteuerten Drehung bewirkt wird, dadurch gekennzeichnet, daß die Innenwand der Elektrode (3) eine Verformung in Form einer Rastkerbe aufweist, in der der Bohrer (6) nach seinem Ausbringen Halt faßt.
